# EUROPÄISCHE PATENTSCHRIFT

(11) **EP 0 920 284 B1**
(45) Veröffentlichungstag und Bekanntmachung des Hinweises auf die Patenterteilung: **05.11.2003**
(21) Anmeldenummer: 97941950.4
(22) Anmeldetag: 21.08.1997
(51) Int. Cl.: A61B 17/80

(54) **FERSENBEIN-KNOCHENPLATTE**
CALCANEAL BONE PLATE
PLAQUE VISSEE POUR LE CALCANEUM

(30) Priorität: 22.08.1996 DE 29614425 U
(43) Veröffentlichungstag der Anmeldung: 09.06.1999
(73) Patentinhaber: Waldemar Link (GmbH & Co.), 22339 Hamburg (DE)
(72) Erfinder: LINK, Helmut, D., D-22397 Hamburg (DE)
(74) Vertreter: Glawe, Delfs, Moll & Partner
(86) Internationale Anmeldenummer: EP9704552
(87) Internationale Veröffentlichungsnummer: WO98007380

(56) Entgegenhaltungen:
- EP-A- 0 253 796
- DE-A- 4 038 082
- DE-U- 9 217 769
- FR-A- 2 622 431

## Beschreibung

Die Erfindung betrifft eine Knochenplatte für die Versorgung von Frakturen des Fersenbeins.

Die komplizierte räumliche Anordnung der Gelenkflächen des Fersenbeins erschwert deren genaue Reposition nach Frakturen. Fortdauernde Beschwerden sind verhältnismäßig häufig (ZWIPP: Der intraartikuläre subthalamische Fersenbeinbruch, Operat. Orthopädie Traumatologie 7 (1995) 237ff.). Zur Stabilisierung der reponierten Fragmente bedient man sich vor allem H- und T-förmiger Platten, die eben sind und ggf. zurecht gebogen werden, um der räumlichen Oberflächenform des Knochens angepaßt zu werden (FR-A-2622431). Dies setzt nicht nur beträchtliches handwerkliches Geschick voraus, sondern auch, daß der Operateur eine detaillierte Vorstellung von der Oberflächenform des betroffenen Knochens hat, die aber anhand des fragmentierten Knochens nur schwer zu gewinnen ist. Auch lassen die zur Verfügung stehenden Umrißformen der H- und T-Platten in vielen Fällen eine hinreichende Anpassung an das Bruchbild vermissen. Unpassende Auswahl oder Gestaltung der Platten hinsichtlich ihres Umrisses und ihrer räumlichen Form kann daher leicht, auch unbemerkt, zu falschen Ergebnissen führen. Auch kann es geschehen, daß infolge zu großer Frakturspalten unzulängliche Heilerfolge erzielt werden.

Der Erfindung liegt die Aufgabe zugrunde, die genannten Nachteile zu verringern. Die erfindungsgemäße Lösung besteht in den Merkmalen des Anspruchs 1 und vorzugsweise denjenigen der Unteransprüche.

Obwohl die vorkommenden Fersenbeinfrakturen sehr vielgestaltig sind (ZWIPP a.a.O.), lassen sich doch bestimmte typische Frakturmuster unterscheiden. Die Erfindung stellt eine für ein häufiges Frakturmuster geeignete Knochenplatte zur Verfügung.

Die erfindungsgemäße Knochenplatte, die lateral anzubringen ist und besonders für sogenannte Tongue-Frakturen geeignet ist, besitzt einen Längsteil mit etwa mittiger Auswölbung, einen von dem posterioren Ende des Längsteils beiderseits vorstehenden Querteil und einen vom anterioren Ende des Längsteils nach unten vorstehenden ersten Vorsprung sowie einen nahe diesem anterioren Ende versetzt zum ersten Vorsprung nach kranial vorstehenden zweiten Vorsprung. Die beiden Vorsprünge sind zueinander (ggf. unter Einbeziehung des Längsteils) um eine etwa parallel zum Längsteil verlaufende Achse abgewinkelt oder gewölbt, so daß der zwischen ihnen befindliche Längsteil eine Aufwölbung erfährt und insbesondere der erste Vorsprung nach innen gezogen ist (Terminologie siehe ZWIPP, a.a.O., S. 238). Der am posterioren Ende des Längsteils befindliche Querteil ist bei Tongue-Brüchen zum Zusammenziehen des posterioren Facettenfragments und des darunter befindlichen tuberositären Fragments geeignet. Der erste Vorsprung kann um den Fersenbeinhals fassen, um den processus anterior zu halten, während der zweite Vorsprung unterhalb der posterioren Facette das posteriore Facettenfragment oder ein an dieser Stelle befindliches weiteres Fragment positioniert.

Die Anpassung an räumliche Oberflächenform des Knochens bezieht sich auf dessen laterale Fläche zwischen dem tuber calcanei und dem processus anterior. Durch die Anpassung der Plattenform an die durchschnittliche Oberflächengestalt des Knochens wird selbst dann, wenn der individuelle Knochen gewisse Abweichungen von der Durchschnittsform zeigen sollte, eine grob fehlerhafte, durch die Form der Knochenplatte bedingte Positionierung der Fragmente ausgeschlossen. Außerdem kann der Operateur in Extremfällen leicht ein wenig nachformen, wobei eine grobe Mißformung nicht zu befürchten ist, weil sich seine Korrekturen auf geringe Formänderungen beschränken können. Durch die komprimierende Ausführung einiger Schraublöcher wird dabei gewährleistet, daß die Fragmente spaltfrei zusammengezogen werden. Dies ist an sich bekannt (DE-U-9217769).

Wenn die Kompressionslöcher an den Enden des Querteils zueinander hin gerichtet sind, werden das posteriore und das tuberositäre Facettenfragment gegeneinander gezogen. Wenn an dem zweiten Vorsprung ein zum Längsteil hin gerichtetes Kompressionsschraubenloch vorgesehen ist, so wird insbesondere der anteriore Bereich des posterioren Facettenfragments gegen das tuberositäre Fragment gezogen. Weist der erste Vorsprung ein zum Längsteil und zum Querteil gerichtetes Kompressionsschraubenloch auf, so wird das sustentakulare Fragment gegen die beiden anderen genannten Fragmente gezogen.

Die Erfindung wird im folgenden näher unter Bezugnahme auf die Zeichnung erläutert, die ein vorteilhaftes Ausführungsbeispiel veranschaulicht. Darin zeigen:
- Fig. 1: eine laterale Ansicht des Fersenbeins mit der erfindungsgemäßen Knochenplatte,
- Fig. 2: eine Ansicht der in Fig.1 gezeigten Knochenplatte in Blickrichtung II, und
- Fig. 3: eine Ansicht der in Fig. 1 gezeigten Knochenplatte in Blickrichting III.

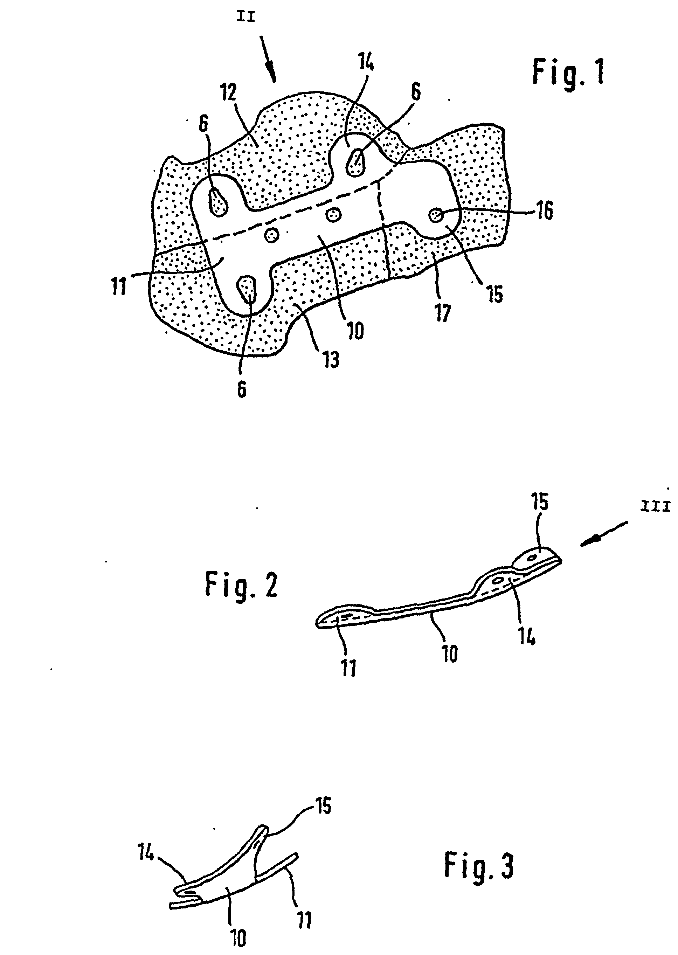

Die in den Fig. 1 bis 3 gezeigte Knochenplatte ist für denjenigen Frakturtyp bestimmt, der in Fig.1 durch Bruchlinien zwischen dem oberen oder posterioren Facettenfragment 12, dem unteren oder tuberositären Fragment 13 und dem sustentakularen Fragment 17 angedeutet sind. Dabei kann insbesondere das posteriore Facettenfragment 12 noch weitere Brüche oder Quetschungen aufweisen. In Fig. 1 ist das rechte Fersenbein in lateraler Ansicht dargestellt (posterior ist auf der linken Seite der Figur, anterior auf der rechten).

Die Knochenplatte weist einen Längsteil 10 auf, an dessen hinterem Ende etwa T-förmig ein Querteil 11 angeordnet ist, der sowohl ober- als auch unterseits über den Längsteil 10 hinausragt und Kompressions-Schraubenlöcher 6 enthält, die zueinander hin gerichtet sind, um ein oberes oder posteriores Facettenfragment 12 mit einem unteren oder tuberositären Fragment zu verbinden und diese Fragmente zusammenzuziehen. Der Längssteil 10 liegt mit seinem hinteren Ende an der Lateralfläche des tuber calcanei und mit seinem vorderen Ende am Hals bzw. an der Lateralfläche des processus anterior. Nahe dem vorderen Ende des Längsteils 10 geht von diesem ein nach oben gerichteter Vorsprung 14 aus, der mit einem Kompressions-Schraubenloch 6 versehen ist und gleichfalls das posteriore Facettenfragment ergreift. Sein Mittenabstand vom vorderen Ende liegt vorzugsweise bei 1-2cm. Am vorderen Ende des Längsteils 10 ist ein Vorsprung 15 nach unten vorgesehen, dessen Schraubloch 16 zum Befestigen des processus anterior dient. Weitere Schraublöcher können über die Länge des Längsteils 10 verteilt sein.

Das im oberen Vorsprung 14 enthaltene Kompressions-Schraubenloch 6 ist nach unten oder ein wenig nach hinten gerichtet.

Der Längsteil 10 ist in seinem mittleren bis vorderen Drittel nach außen gewölbt. Außerdem sind seine oberen und unteren Vorsprünge 14, 15 um eine parallel zu seiner Längsrichtung verlaufende Achse nach außen gewölbt, damit der untere Vorsprung 15 um so besser um den Hals des Fersenbeins fassen kann. Sie sind auch ein wenig gegenüber dem hinteren Querteil 11 verwunden, wie Fig.3 zeigt.

Ein Vorteil dieser Knochenplatte mit ihren ziemlich kurzen Vorsprüngen besteht darin, daß sie zwar in Richtung auf angrenzende Gelenkteile (z.B. zum Talus) hin ausgerichtet sind, aber durch ihre kurze Ausführung nicht störend in den Gelenkbereich hineinragen.

## Patentansprüche

1. Knochenplatte für die Versorgung von Frakturen des Fersenbeins mit einem Längsteil (10), der ein vorderes und ein hinteres Ende aufweist, und einem zumindest zu einer Seite vorstehenden Querteil (11), an denen Schraubenlöcher vorgesehen sind, **dadurch gekennzeichnet, daß** der Längsteil (10) eine Auswölbung im mittleren bis vorderen Drittel aufweist, daß der Querteil (11) von dem hinteren Ende des Längsteils (10) beiderseits vorsteht, daß sie einen vom vorderen Ende des Längsteils (10) nach einer Kaudalen Seite vorstehenden ersten Vorsprung (15) zum Halten des processus anterior und einen nahe diesem vorderen Ende versetzt zum ersten Vorsprung zur Kranialen Seite hin vorstehenden zweiten Vorsprung (14) zum Ansatz unterhalb der posterioren Facette aufweist, wobei die beiden Vorsprünge (14, 15) gegeneinander um eine im wesentlichen parallel zum Längsteil (10) verlaufende Achse abgewinkelt oder gewölbt sind, und daß der Querteil (11) und/oder mindestens einer der Vorsprünge (14, 15) Kompressions-Schraubenlöcher (6) enthalten.

2. Knochenplatte nach Anspruch 1, **dadurch gekennzeichnet, daß** die Kompressions-Schraubenlöcher (6) zumindest in den Enden des Querteils (11) vorgesehen sind und eine zueinander gerichtete Richtungskomponente aufweisen.

3. Knochenplatte nach Anspruch 1, **dadurch gekennzeichnet, daß** ein Kompressions-Schraubenloch (6) zumindest in dem zweiten Vorsprung (14) vorgesehen ist und eine zum Längsteil (10) hin gerichtete Richtungskomponente aufweist.

4. Knochenplatte nach Anspruch 1, **dadurch gekennzeichnet, daß** ein Kompressions-Schraubenloch (16) zumindest in dem ersten Vorsprung (15) vorgesehen ist und zum Längsteil (10) und zum Querteil (11) gerichtete Richtungskomponenten aufweist.

## Claims

1. A bone plate for the treatment of fractures of the calcaneus, having a longitudinal part (10) which has a front end and a rear end, and having a transverse part (11) projecting at least on one side and in which screw holes are provided, **characterised in that** the longitudinal part (10) has a domed portion in its middle to front third, **in that** the transverse part (11) projects on either side from the rear end of the longitudinal part (10), **in that** it has a first projection (15), which protrudes on one caudal side from the front end of the longitudinal part (10), for retaining the anterior process, and has a second projection (14), which protrudes near thereto offset in relation to the first projection on the cranial side, for application below the posterior facet, wherein the two projections (14,15) are offset or curved with respect to one another about an axis extending substantially parallel to the longitudinal part (10), and **in that** the transverse part (11) and/or at least one of the projections (14,15) include compression screw holes (6).

2. A bone plate according to Claim 1, **characterised in that** the compression screw holes (6) are provided at least in the ends of the transverse part (11) and have a directional component directed towards one another.

3. A bone plate according to Claim 1, **characterised in that** one compression screw hole (6) is provided at least in the second projection (14) and has a directional component directed towards the longitudinal part (10).

4. A bone plate according to Claim 1, **characterised in that** one compression screw hole (6) is provided at least in the first projection (15) and has directional components directed towards the longitudinal part (10) and towards the transverse part (11).

## Revendications

1. Plaque vissée pour soigner des fractures du calcanéum, comprenant un élément longitudinal (10) présentant une extrémité avant et une extrémité arrière, et un élément transversal (11) saillant au moins vers un côté, sur lesquels des trous pour vis sont prévus, **caractérisée en ce que** l'élément longitudinal (10) présente un bombement dans le tiers central jusqu'au tiers avant, **en ce que** l'élément transversal (11) saille des deux côtés de l'extrémité arrière de l'élément longitudinal (10), **en ce qu'**elle présente une première saillie (15) saillant de l'extrémité avant de l'élément longitudinal (10) vers un côté caudal pour maintenir le processus antérieur, et une deuxième saillie (14) saillant vers le côté cranial, à proximité de cette extrémité avant et décalée par rapport à la première saillie, pour être placée sous la facette postérieure, les deux saillies (14, 15) étant coudées ou bombées l'une par rapport à l'autre autour d'un axe s'étendant pour l'essentiel parallèlement à l'élément longitudinal (10), et **en ce que** l'élément transversal (11) et/ou au moins l'une des saillies (14, 15) comprend des trous pour vis de compression (6).

2. Plaque vissée selon la revendication 1, **caractérisée en ce que** les trous pour vis de compression (6) sont prévus au moins dans les extrémités de l'élément transversal (11) et présentent des composantes de direction orientées l'une vers l'autre.

3. Plaque vissée selon la revendication 1, **caractérisée en ce qu'**un trou pour vis de compression (6) est prévu au moins dans la deuxième saillie (14) et présente une composante de direction orientée vers l'élément longitudinal (10).

4. Plaque vissée selon la revendication 1, **caractérisée en ce qu'**un trou pour vis de compression (16) est prévu au moins dans la première saillie (15) et présente des composantes de direction orientées vers l'élément longitudinal (10) et vers l'élément transversal (11).
